# EUROPÄISCHE PATENTSCHRIFT

(11) **EP 1 255 722 B1**
(45) Veröffentlichungstag und Bekanntmachung des Hinweises auf die Patenterteilung: **17.01.2007**
(21) Anmeldenummer: 01923583.7
(22) Anmeldetag: 16.02.2001
(51) Int. Cl.: C07C 67/08, C07C 69/54

(54) **VERFAHREN ZUR HERSTELLUNG VON ESTERN alpha, beta -UNGESÄTTIGTER CARBONSÄUREN**
METHOD FOR PRODUCING ESTERS OF alpha, beta -UNSATURATED CARBOXYLIC ACIDS
PROCEDE POUR LA PRODUCTION D'ESTERS D'ACIDES CARBOXYLIQUES alpha, beta -INSATURES

(30) Priorität: 17.02.2000 DE 10007213
(43) Veröffentlichungstag der Anmeldung: 13.11.2002
(73) Patentinhaber: BASF Aktiengesellschaft, 67056 Ludwigshafen (DE)
(72) Erfinder: HERZOG, Reinhard, 67063 Ludwigshafen (DE); NESTLER, Gerhard, 1070 Wien (AT); SCHRÖDER, Jürgen, 67071 Ludwigshafen (DE)
(74) Vertreter: Pohl, Michael Friedrich
(86) Internationale Anmeldenummer: PCT/EP2001/001754
(87) Internationale Veröffentlichungsnummer: WO 2001/060779

(56) Entgegenhaltungen:
- DE-A- 19 536 191

## Beschreibung

Die Erfindung betrifft ein Verfahren zur Herstellung von Estern einer α,β-ungesättigten Carbonsäure durch Umsetzung der Carbonsäure mit einem Alkanol in Gegenwart eines sauren Veresterungskatalysators in Gegenwart eines Schleppmittels, mit dem das während der Veresterung gebildete Reaktionswasser aus dem Veresterungsverfahren entfernt wird.

Die Herstellung von Alkylestern α,β-ungesättigter Carbonsäuren ist allgemein bekannt. Sie wird üblicherweise durch Verestern der Säuren mit dem entsprechenden Alkohol bei erhöhter Temperatur in flüssiger Phase mit oder ohne Lösungsmittel und in Gegenwart einer starken Säure als Katalysator durchgeführt (siehe z. B.
DE-A-23 39 519). Zur Vermeidung einer Polymerisation werden meist Polymerisationsinhibitoren zugegeben. Von besonderer technischer Bedeutung ist die Veresterung von (Meth)acrylsäure. Der Begriff (Meth)acrylsäure bezeichnet in bekannter Weise Acryl- oder Methacrylsäure.

Nachteilig an den Veresterungsverfahren des Standes der Technik ist, dass sich unter den Veresterungsbedingungen als Nebenreaktionen noch nicht umgesetzter Ausgangsalkohol sowie noch nicht umgesetzte Carbonsäure an die Doppelbindung von bereits gebildetem Carbonsäurealkylester addiert (Michael-Addition). Auch eine Mehrfachaddition ist möglich. Ferner können gemischte Typen auftreten. Diese Addukte sind Alkoxyester und Acyloxyester, und man bezeichnet sie kurz als Oxyester. Charakteristisch für die Oxyester ist, dass ihr Siedepunkt oberhalb der Siedepunkte von Ausgangssäure, Ausgangsalkohol, gebildetem Zielester sowie gegebenenfalls mitverwendetem organischen Lösungsmittel liegt.
Bei der Gewinnung des gewünschten Esters fallen sie daher als Rückstand an und bedingen beträchtliche Ausbeuteverluste. Daher sind zahlreiche Versuche unternommen worden, aus den Oxyestern wenigstens einen Teil der eingesetzten Verbindungen oder den Zielester zurückzugewinnen, siehe z. B. die DE-A- 195 36 191 und den darin aufgeführten Stand der Technik.

Bei dem Rückspaltungsverfahren der DE 195 36 191 wird eine beträchtliche Menge an Olefinen gebildet, die in dem Veresterungsverfahren nicht weiterverwendet werden kann und daher abgetrennt und ausgeschleust werden muss.

Ein weiterer Nachteil der üblichen Veresterungsverfahren ist eine Folge der Tatsache, dass der Esterbildung eine Gleichgewichtsreaktion zugrunde liegt. Um wirtschaftliche Umsätze zu erzielen, wird in der Regel ein Einsatzstoff im Überschuss eingesetzt und/oder das gebildete Veresterungswasser und/oder der Zielester werden aus dem Gleichgewicht entfernt. Um das Gleichgewicht in Richtung der Esterbildung zu verschieben, wird häufig ein organisches Schleppmittel zugegeben, das mit Wasser ein Azeotrop bildet. Vor allem die Veresterung mit höheren Alkanolen wird in Gegenwart eines Schleppmittels für das Reaktionswasser durchgeführt (Kirk-Othmer, "Encyclopedia of Chemical Technology", Vol. 1, S. 347, Ullmann's Encyclopedia of Industrial Chemistry, 5th Ed., 1985, Vol. A1, 168; US 2 917 538, US 5 386 052). Vorzugsweise werden inerte Kohlenwasserstoffe, z. B. Cyclohexan, Hexan, Benzol und Toluol eingesetzt.

Der Zusatz eines zusätzlichen "fremden" Lösungsmittels ist aber nachteilig, da dieses separat abgetrennt und vor der Rückführung gegebenenfalls gereinigt werden muss.

Die DE 2 548 561 schlägt daher vor, das bei der Umsetzung von Acrylsäure mit Ethylhexanol gebildete Reaktionswasser als Azeotrop mit Ethylhexanol abzutrennen. Gemäß dem Ausführungsbeispiel wird Ethylhexanol im Überschuss zu Acrylsäure (1:1,42) eingesetzt. Nachteilig dabei ist, dass die Veresterung unter Vakuum erfolgen muss und ein Rückstand von ca. 8 Gew.-%, bezogen auf den Zielester, anfällt, der entsorgt werden muss. Das Verfahren ist damit umweltbelastend und unwirtschaftlich.

Die Beeinflussung des Veresterungsgleichgewichts durch den Einsatz eines größeren Überschusses an einem Ausgangsstoff (Alkanol oder Carbonsäure) führt zur Bildung von Nebenprodukten, wie z. B. Ether und Olefine aus dem verwendeten Alkanol sowie Acyloxyester und Alkoxyester durch Michael-Addition des Alkanols bzw. der Carbonsäure. Dies ist z. B. für Acrylsäure beschrieben in US-A-4 280 010 und in DE-A-2 339 519. Diese Nebenprodukte müssen aufwendig abgetrennt und entsorgt werden, was unwirtschaftlich ist und die Umwelt belastet.

Auch ohne den Einsatz eines größeren Überschusses an Alkohol werden unter den stark sauren Veresterungsbedingungen die Ester und der Alkohol zum Teil wieder gespalten und Olefine gebildet, wie z. B. in der DE-A-195 36 191 und Houben-Weyl, Methoden der Organischen Chemie, Band VIII/3, 1952, S. 534, beschrieben ist.

Die meisten Verfahren des Standes der Technik haben also den Nachteil gemeinsam, dass unerwünschte Nebenprodukte abgetrennt werden müssen, und dass ein zusätzliches Lösungsmittel als Schleppmittel zur Entfernung des Veresterungswassers benötigt wird.

Der Erfindung liegt daher die Aufgabe zugrunde, ein Veresterungsverfahren bereitzustellen, das einfacher durchführbar ist und bei dem die Bildung unerwünschter Nebenprodukte verringert wird.

Überraschenderweise wurde nun gefunden, dass diese Aufgabe gelöst wird, wenn man als Schleppmittel das Olefin verwendet, das durch Wasserabspaltung aus dem zur Veresterung eingesetzten Alkanol entsteht.

Die Erfindung betrifft daher ein Verfahren zur Herstellung eines Esters einer α,β-ungesättigten Carbonsäure durch Umsetzung der Carbonsäure mit einem Alkohol, der ausgewählt ist unter C₆-C₁₂-Alkanolen, Cyclopentanol und Cyclohexanol, in Gegenwart eines sauren Veresterungskatalysators und eines Schleppmittels zur Entfernung des bei der Veresterung gebildeten Reaktionswassers, wobei als Schleppmittel das dem Alkohol zugrunde liegende Olefin verwendet wird, d. h. es wird ein Olefin verwendet, das einem durch Wasserabspaltung aus dem eingesetzten Alkohol erhältlichen Olefin entspricht.

Mit Ausnahme des verwendeten Schleppmittels wird das erfindungsgemäße Veresterungsverfahren in konventioneller Weise durchgeführt. Geeignete Verfahren sind z. B. beschrieben in Ullmann's Encyclopedia of Industrial Chemistry, 5th Ed., 1985, Vol. A1, 168, 169; Kirk-Othmer, Encyclopedia of Chemical Technology, Vol. 1, S. 341 - 348; US-A-2 917 538; US-A-5 385 052. Die Verfahren sind am Beispiel von Acrylsäure beschrieben, das erfindungsgemäße Verfahren ist jedoch nicht auf Acrylsäure beschränkt. Die Ester anderer α,β-ungesättigter Carbonsäuren können analog hergestellt werden.

Die Veresterungen werden typischerweise durchgeführt bei Temperaturen zwischen 80 und 160 °C, vorzugsweise 90 bis 130 °C, und in Gegenwart eines sauren Veresterungskatalysators, z. B. einer Mineralsäure, einer Sulfonsäure oder Phosphorsäure. Besonders gut geeignet sind Schwefelsäure und Sulfonsäuren, insbesondere p-Toluol-, Benzol-, Dodecylbenzol- und Methansulfonsäure. Die Katalysatormenge beträgt 0,1 bis 10 Gew.-%, vorzugsweise 0,5 bis 5 Gew.-%, bezüglich der übrigen Einsatzstoffe. Die Veresterung wird normalerweise auch in Gegenwart eines Inhibitors durchgeführt, der die Polymerisation der Carbonsäure und/oder des Esters hemmt. Besonders geeignete Inhibitoren sind Hydrochinon, Hydrochinonmonomethylether, p-Methoxyphenol, p-Benzochinon, Phenothiazin, 4-Hydroxy-2,2,6,6-tetramethyl-1-oxyl-piperidin und Methylenblau, die zur Stabilisierung in Mengen von 200 bis 2 000 ppm, bezogen auf das Gewicht der Einsatzstoffe, verwendet werden. Das erfindungsgemäße Verfahren ist allerdings nicht auf spezielle Katalysatoren oder Inhibitoren beschränkt, und ebensowenig spielen die Mengenverhältnisse von Säure:Alkohol, der bei der Veresterung herrschende Druck oder die Reaktionszeit eine wesentliche Rolle. Ein typisches Mol-Verhältnis von Alkohol:Säure ist 1:0,7 bis 1,2, und typische Reaktionszeiten sind 1 bis 10 Stunden, vorzugsweise 1 bis 6 Stunden. Die Veresterung kann drucklos, mit Überdruck oder Unterdruck und sowohl kontinuierlich als auch diskontinuierlich durchgeführt werden.

Als Apparatur zur Durchführung der Veresterung und Abtrennung des Zielesters aus dem Reaktionsgemisch sind gängige Anlagen geeignet, wie sie z. B. beschrieben sind in Ullmann's Encyclopedia of Industrial Chemistry, 5th Ed., 1985, Band A1, S. 168, 169 und Kirk-Othmer "Encyclopedia of Chemical Technology", Vol. 1, S. 341, 342.

Alkohol, Säure, Katalysator und gewünschtenfalls Polymerisationsinhibitor werden kontinuierlich oder chargenweise in einen Reaktor, beispielsweise einen einfachen beheizbaren Kessel, eingefüllt.

Das dem verwendeten Alkohol zugrunde liegende Olefin (oder die zugrunde liegenden Olefine) wird (werden), wie erwähnt, als Nebenprodukt bei der Veresterung und der Aufarbeitung gebildet.

Die Menge an gebildetem Olefin ist abhängig von der Reaktionsführung und den Ausgangssubstanzen. Im kontinuierlichen Betrieb wird die Menge an Olefin gegebenenfalls durch Ausschleusen konstant gehalten.

Bei der Bildung der Olefine durch Wasserabspaltung aus dem zur Veresterung verwendeten Alkanol, durch Spaltung des Esters unter den Veresterungsbedingungen oder durch Rückspaltung der Oxyester bildet sich in vielen Fällen nicht nur ein Olefin, sondern durch Isomerisierung ein Gemisch von zwei oder mehreren Olefinen, je nach eingesetztem Alkohol. Beispielsweise kommt es bei Verwendung von 2-Ethylhexan-1-ol als Alkohol nicht nur zur Bildung von 2-Ethylhexen-1, sondern zur Bildung eines Gemisches aus isomeren Octenen, das 2-Ethylhexen-2 und 3-Methylhepten-2 als Hauptkomponenten enthält. Der Anteil der Komponenten hängt ab von den Reaktionsbedingungen.

Dementsprechend ist erfindungsgemäß unter dem Ausdruck "das dem Alkohol zugrunde liegende Olefin" nicht nur ein (einziges) Olefin zu verstehen, sondern auch ein Gemisch von zwei oder mehreren isomeren Olefinen.

Das aus dem Reaktor abdestillierte azeotrope Gemisch mit dem Olefin als Schleppmittel kann auch gewisse Mengen an Zielester und/oder Ausgangsalkohol und/oder Ausgangssäure enthalten. Die Mengen sind abhängig von der Reaktionsführung und der Art der verwendeten Säure und des verwendeten Alkohols. Aus dem abdestillierten azeotropen Gemisch scheidet sich das Wasser ab und wird abgetrennt. Die organische Phase, die das Olefin enthält, kann zusammen mit den übrigen Bestandteilen des Gemischs, falls vorhanden, direkt wieder der Veresterungsreaktion zugeführt werden. Diese Zuführung kann kontinuierlich oder chargenweise erfolgen. Das abgeschiedene Wasser kann noch einer Aufarbeitung unterzogen werden, um etwa darin enthaltenen Zielester zu gewinnen und/oder Ausgangsverbindungen zurückzugewinnen, die der Veresterungsreaktion wieder zugeführt werden können. Außerdem kann die Wasserphase bei einem der gegebenenfalls vorgesehenen Waschprozesse bei der Aufarbeitung des Veresterungsgemisches als Waschflüssigkeit eingesetzt werden. Wenn gewünscht, kann auch die Olefin-Phase behandelt werden, um darin enthaltenen Ester oder sonstige Produkte zu entfernen, bevor das Olefin wieder dem Veresterungs-Reaktor zugeführt wird. Am einfachsten und wirtschaftlichsten und daher bevorzugt ist es jedoch, das Schleppmittel samt darin enthaltenen Beimengungen, wie Zielester und/oder Ausgangsalkohol und/oder Ausgangssäure, gegebenenfalls auch mit sonstigen Nebenprodukten, direkt wieder dem Veresterungs-Reaktor zuzuführen.

Die Aufarbeitung des Veresterungs-Reaktionsgemischs bei dem erfindungsgemäßen Verfahren zum Verestern einer α,β-ungesättigten Carbonsäure erfolgt in konventioneller Weise, d. h. nicht umgesetzte Ausgangsverbindungen sowie der Zielester werden vom Reaktionsgemisch destillativ abgetrennt, wobei der zur Veresterung verwendete Säurekatalysator gegebenenfalls vorher durch Extraktion mittels Wasser und/oder wässriger Lauge abgetrennt wird (vergleiche z. B. Ullmann's Encyclopedia of Industrial Chemistry, Vol. A1, 5th Ed., VCH, S. 167ff). Die Aufarbeitung kann nach Beendigung der Veresterungsreaktion erfolgen, es kann aber auch kontinuierlich Reaktionsgemisch abgezogen und aufgearbeitet werden. Das bei der destillativen Aufarbeitung des Veresterungs-Reaktionsgemisches verbleibende Sumpfprodukt enthält die Oxyester. Das Sumpfprodukt kann einer Behandlung zur Rückgewinnung von Ausgangssubstanzen und/oder zur Gewinnung von Zielester unterzogen werden, wobei bevorzugt das in DE-A-195 36 191 beschriebene Verfahren angewendet wird. Das dabei erhaltene Olefin kann als Schleppmittel eingesetzt werden. Die Spaltprodukte werden bevorzugt in den Veresterungs-Reaktor zurückgeführt.

Bei dem Verfahren gemäß DE 195 36 191 wird das zu spaltende Sumpfprodukt kontinuierlich oder diskontinuierlich aus der destillativen Aufarbeitung des Veresterungsgemischs ausgeschleust und mit dem Spaltkatalysator dem Spaltreaktor zugeführt. Auch eine halbkontinuierliche Reaktionsführung ist möglich, bei der das zu spaltende Produkt kontinuierlich dem Spaltreaktor, der den Spaltkatalysator enthält, zugeführt wird und das Sumpfprodukt erst nach Beendigung der Spaltung aus dem Spaltreaktor batchweise entfernt wird. Die Spaltprodukte werden kontinuierlich destillativ abgetrennt. Die Spaltung erfolgt bevorzugt in Anwesenheit von Säure bei einer Temperatur von etwa 150 bis 250 °C sowie bei einem solchen Druck, dass die aus den Oxyestern entstehenden Spaltprodukte abdampfen. Bevorzugt wird das Verfahren im Beisein von molekularem Sauerstoff durchgeführt. Bezüglich der genauen Durchführung des Verfahrens wird auf die DE-A-19 536 191 Bezug genommen.

Die Spaltprodukte enthalten neben dem Zielester, Ausgangscarbonsäure und Ausgangsalkohol einen beträchtlichen Anteil an Olefinen, z. B. bis zu 20 Gew.-% Octene bei der Spaltung des Rückstands aus der Herstellung von 2-Ethylhexylacrylat. Bei diesen Octenen handelt es sich im Wesentlichen um ein Gemisch aus isomeren Octenen mit 2-Ethylhexen-2 und 3-Methylhepten-2 als Hauptkomponenten.

Das bei der Oxyester-Spaltung entstehende Olefin kann kontinuierlich oder diskontinuierlich dem Veresterungsverfahren zugeführt werden. Eine vorherige Abtrennung der weiteren Oxyester-Spaltprodukte, nämlich Zielester, Ausgangsalkohol und Ausgangssäure, ist nicht erforderlich, kann aber durchgeführt werden. Eine Möglichkeit ist es, aus dem bei der Oxyester-Spaltung abdampfenden, die Spaltprodukte enthaltenden Gemisch den monomeren Ester abzutrennen und die restlichen Spaltprodukte, d. h. Olefin, Ausgangssäure und Ausgangsalkohol, und gegebenenfalls weitere Spaltprodukte in den Veresterungsreaktor zurückzuführen. Dadurch wird der monomere Ester einer möglichen Weiterreaktion entzogen, die zurückgewonnenen Ausgangsprodukte werden zwecks Ausbeuteerhöhung der Reaktion wieder zugeführt, und das Olefin findet als Schleppmittel für Reaktionswasser Verwendung.

Das erfindungsgemäße Verfahren ist nicht auf bestimmte α,β-ungesättigte Carbonsäuren beschränkt, sondern es kommen einfach oder mehrfach ungesättigte Carbonsäuren sowie Mono- oder Dicarbonsäuren in Betracht. Bevorzugt angewendet wird das erfindungsgemäße Verfahren jedoch bei einfach ungesättigten Monocarbonsäuren mit 3 bis 6 Kohlenstoffatomen und bei einfach ungesättigten Dicarbonsäuren mit 4 bis 8 Kohlenstoffatomen, wie z. B. Acrylsäure, Crotonsäure, Malein- und Fumarsäure, Itaconsäure. Mit besonderem Vorteil wird das erfindungsgemäße Verfahren bei Acrylsäure und Methacrylsäure und insbesondere zur Herstellung von 2-Ethylhexylacrylat angewandt.

Bevorzugte Alkanole sind solche mit 6 bis 10 Kohlenstoffatomen und insbesondere 2-Ethylhexanol (2-Ethylhexan-1-ol) oder 2-Propylheptanol.

Es hat sich überraschenderweise gezeigt, dass Olefin-Isomerengemische trotz eigentlich zu erwartender Bildung von Estern als Schleppmittel eingesetzt werden können, ohne dass dadurch die Veresterung und die Qualität des Zielesters beeinflusst wird. Ein typisches Beispiel für ein solches Isomerengemisch ist das genannte Gemisch aus isomeren Octenen mit 2-Ethylhexen-2 und 3-Methylhepten-2 als Hauptkomponenten.

Das erfindungsgemäße Verfahren hat den Vorteil, dass es einfacher durchführbar ist, weil kein "fremdes" Schleppmittel verwendet wird, das eine zusätzliche Komponente in das System einbringt. Vielmehr wird als Schleppmittel ein Olefin verwendet, das sich bei der Veresterung ohnehin als Nebenprodukt bildet. Eine eigene Abtrennung dieses Nebenprodukts, das leichter siedet als der gewünschte Ester, ist daher nicht mehr erforderlich.

Die Erfindung wird im Folgenden anhand von Beispielen veranschaulicht, die jedoch nur der Erläuterung dienen und in keiner Weise beschränkend zu verstehen sind:

Die in den folgenden Beispielen eingesetzte Acrylsäure enthielt im Wesentlichen folgende Komponenten:

| | |
|---|---|
| Acrylsäure | 99,7 % |
| Essigsäure | 0,10 % |
| Propionsäure | 0,04 % |
| Diacrylsäure | 0,08 % |
| Wasser | 0,05 % |
| MEHQ (Hydrochinonmonomethylether) | 0,02 % |

Als Schleppmittel wurde ein Octengemisch verwendet, das bei der Spaltung der bei der Herstellung von 2-Ethylhexylacrylat anfallenden Oxyester stammte. Es enthielt entsprechend der gaschromatographischen Analyse folgende Hauptkomponenten:

| | |
|---|---|
| 2-Ethylhexen-2 | 32 % |
| 3-Methylhepten-2 | 46,7 % |
| 5-Methylhepten-2 | 14,3 % |

### Beispiel 1

In einem Rührreaktor wurden 336 g eines Octen-Gemisches, 238 g Acrylsäure, 1,2 g Phenothiazin und 5,74 g Schwefelsäure (98 %ig) unter Rühren erhitzt und 5 Stunden bei 114 - 117 °C gehalten. Nach der Neutralisation des Reaktionsgemischs konnten mittels Gaschromatographie keine Octylacrylate nachgewiesen werden.

### Beispiel 2

In einem Rührreaktor mit aufgesetzter Destillationskolonne (30 cm, 0,5 cm-Raschigringe), Kondensator und Wasserabscheider wurde ein Gemisch aus 238 g Acrylsäure, 390 g 2-Ethylhexanol, 190 g Octen-Gemisch, 0,5 g Phenothiazin und 6,28 g Schwefelsäure (98 %ig) unter Rühren und unter Normaldruck auf Siedetemperatur erhitzt und 1,5 Stunden unter Rückfluss gehalten. Die Temperatur im Reaktor stieg von 116°C auf 159 °C. Über den Wasserabscheider wurden dabei 50 g Wasser abgetrennt. Der Rest-Acrylsäuregehalt im Reaktionsproduktgemisch betrug 3,1 %. Der Acrylsäureumsatz betrug 99 % der Theorie. Die gaschromatographische Analyse zeigte, dass keine isomeren Octylester gebildet wurden.

### Beispiel 3

Es wurde wie im Beispiel 2 verfahren, jedoch anstelle des Octen-Gemischs wurde Cyclohexan eingesetzt. Die notwendige Reaktionsdauer betrug 2,5 Stunden. Die Reaktionstemperatur stieg von 98°C auf 114 °C. Der Acrylsäuregehalt des Endgemischs betrug 3,2 %, der Acrylsäureumsatz 99 % der Theorie.

## Patentansprüche

1. Verfahren zur Herstellung eines Esters einer α,β-ungesättigten Carbonsäure durch Umsetzung der Carbonsäure mit einem Alkohol, der ausgewählt ist unter C₆-C₁₂-Alkanolen, Cyclopentanol und Cyclohexanol, in Gegenwart eines sauren Veresterungskatalysators und eines Schleppmittels zur Entfernung des bei der Veresterung gebildeten Reaktionswassers, **dadurch gekennzeichnet, dass** als Schleppmittel ein Olefin verwendet wird, dass einem durch Wasserabspaltung aus dem eingesetzten Alkohol erhältlichen Olefin entspricht.

2. Verfahren nach Anspruch 1, **dadurch gekennzeichnet, dass** Olefin und Wasser destillativ als Gemisch aus dem Reaktionsgemisch entfernt werden, aus dem Gemisch Wasser abgetrennt und das Olefin in das Veresterungsverfahren zurückgeführt wird.

3. Verfahren nach Anspruch 1 oder 2, **dadurch gekennzeichnet, dass** nach beendeter Reaktion aus dem Reaktionsgemisch die flüchtigen Bestandteile abgetrennt werden und das zurückbleibende, Oxyester enthaltende Produkt aufgearbeitet wird, wobei die Oxyester gespalten werden und das Spaltprodukt, gegebenenfalls nach Abtrennung des im Spaltprodukt enthaltenen Esters, als Schleppmittel in das Veresterungsverfahren zurückgeführt wird.

4. Verfahren nach einem der Ansprüche 1 bis 3, **dadurch gekennzeichnet, dass** als Alkohol ein lineares oder verzweigtes C₆-C₁₀-Alkanol verwendet wird.

5. Verfahren nach Anspruch 4, **dadurch gekennzeichnet, dass** als Alkohol 2-Ethylhexanol-1 oder 2-Propylheptanol verwendet wird.

6. Verfahren nach Anspruch 5, **dadurch gekennzeichnet, dass** als Schleppmittel ein Gemisch aus isomeren Octenen mit 2-Ethylhexen-2 und 3-Methylhepten-2 als Hauptkomponenten verwendet wird.

7. Verfahren nach einem der Ansprüche 1 bis 6, **dadurch gekennzeichnet, dass** als α,β-ungesättigte Carbonsäure eine einfach ungesättigte Monocarbonsäure mit 3 bis 6 Kohlenstoffatomen verwendet wird.

8. Verfahren nach einem der Ansprüche 1 bis 6, **dadurch gekennzeichnet, dass** als α,β-ungesättigte Carbonsäure eine einfach ungesättigte Dicarbonsäure mit 4 bis 8 Kohlenstoffatomen verwendet wird.

9. Verfahren nach Anspruch 7, **dadurch gekennzeichnet, dass** als α,β-ungesättigte Carbonsäure Acrylsäure oder Methacrylsäure verwendet wird.

## Claims

1. A process for the preparation of an ester of an α,β-unsaturated carboxylic acid by reacting the carboxylic acid with an alcohol which is selected from C₆-C₁₂-alkanols, cyclopentanol and cyclohexanol, in the presence of an acidic esterification catalyst and of an entraining agent for removing the water of reaction formed in the esterification, wherein the entraining agent used is the olefin which corresponds to an olefin obtainable by eliminating water from the alcohol employed.

2. A process as claimed in claim 1, wherein olefin and water are removed from the reaction mixture as a mixture by distillation, water is separated off from the mixture and the olefin is recycled to the esterification process.

3. A process as claimed in claim 1 or 2, wherein, after the end of the reaction, the volatile components are separated off from the reaction mixture and the remaining, oxyester-containing product is worked up, the oxyesters being cleaved and the cleavage product, if necessary after removal of the ester contained in the cleavage product, being recycled as entraining agent to the esterification process.

4. A process as claimed in any of claims 1 to 3, wherein the alcohol used is a linear or branched C₆-C₁₀-alkanol.

5. A process as claimed in claim 4, wherein the alcohol used is 2-ethylhexan-1-ol or 2-propylheptanol.

6. A process as claimed in claim 5, wherein the entraining agent used is a mixture of isomeric octenes containing 2-ethylhex-2-ene and 3-methylhept-2-ene as main components.

7. A process as claimed in any of claims 1 to 6, wherein the α,β-unsaturated carboxylic acid used is a monounsaturated monocarboxylic acid of 3 to 6 carbon atoms.

8. A process as claimed in any of claims 1 to 6, wherein the α,β-unsaturated carboxylic acid used is a monounsaturated dicarboxylic acid of 4 to 8 carbon atoms.

9. A process as claimed in claim 7, wherein the α,β-unsaturated carboxylic acid used is acrylic acid or methacrylic acid.

## Revendications

1. Procédé de préparation d'un ester d'un acide carboxylique α,β-insaturé par réaction de l'acide carboxylique avec un alcool qui est choisi parmi des alcanols en C₆-C₁₂, du cyclopentanol et du cyclohexanol, en présence d'un catalyseur d'estérification acide et d'un agent d'entraînement pour éliminer l'eau réactionnelle formée au cours de l'estérification, **caractérisé en ce que**, comme agent d'entraînement, on utilise une oléfine qui correspond à une oléfine que l'on peut obtenir par élimination d'eau à partir de l'alcool mis en oeuvre.

2. Procédé suivant la revendication 1, **caractérisé en ce que** l'oléfine et l'eau sont éliminées par distillation hors du mélange réactionnel, sous la forme d'un mélange, l'eau étant séparée du mélange et l'oléfine étant recyclée dans le procédé d'estérification.

3. Procédé suivant la revendication 1 ou 2,
**caractérisé en ce que**, une fois la réaction achevée, on sépare du mélange réactionnel les éléments volatils et on traite le produit subsistant, contenant des oxyesters, les oxyesters étant coupés et le produit de coupure étant, éventuellement après séparation de l'ester contenu dans le produit de coupure, recyclé comme agent d'entraînement dans le procédé d'estérification.

4. Procédé suivant l'une des revendications 1 à 3,
**caractérisé en ce que**, comme alcool, on utilise un alcanol en C₆-C₁₀ linéaire ou ramifié.

5. Procédé suivant la revendication 4, **caractérisé en ce que**, comme alcool, on utilise du 2-éthylhexanol-1 ou du 2-propylheptanol.

6. Procédé suivant la revendication 5, **caractérisé en ce que**, comme agent d'entraînement, on utilise un mélange d'octènes isomères avec du 2-éthylhexène-2 et du 3-méthylheptène-2, comme composants principaux.

7. Procédé suivant l'une des revendications 1 à 6, **caractérisé en ce que**, comme acide carboxylique α,β-insaturé, on utilise un acide monocarboxylique comportant 3 à 6 atomes de carbone, insaturé une seule fois.

8. Procédé suivant l'une des revendications 1 à 6, **caractérisé en ce que**, comme acide carboxylique α,β-insaturé, on utilise un acide dicarboxylique comportant 4 à 8 atomes de carbone, insaturé une seule fois.

9. Procédé suivant la revendication 7, **caractérisé en ce que**, comme acide carboxylique α,β-insaturé, on utilise de l'acide acrylique ou de l'acide méthacrylique.
